# EUROPEAN PATENT SPECIFICATION

(11) **EP 1 570 943 B1**
(45) Date of publication and mention of the grant of the patent: **08.05.2013**
(21) Application number: 05251248.0
(22) Date of filing: 02.03.2005
(51) Int. Cl.: B23K 35/32, B23K 35/00, C04B 37/02, A61N 1/00, B23K 103/18

(54) **Layered deposition braze material**
Geschichtetes abgelagertes Hartlotmaterial
Matériau de brasage sous forme de dépot en couches

(30) Priority: 03.03.2004 US 793006; 03.03.2004 US 793457
(43) Date of publication of application: 07.09.2005
(73) Proprietor: The Alfred E Mann Foundation for Scientific Research, Santa Clarita, CA 91380-9005 (US)
(72) Inventor: Schnittgrund, Gary, Granada Hills, California 91344 (US)
(74) Representative: Carter, Stephen John

(56) References cited:
- GB-A- 813 829
- US-A- 3 897 223
- US-A1- 2002 192 481
- US-B1- 6 221 513
- PATENT ABSTRACTS OF JAPAN vol. 018, no. 381 (C-1226), 18 July 1994 (1994-07-18) & JP 06 105899 A (KYOCERA CORP), 19 April 1994 (1994-04-19)
- PATENT ABSTRACTS OF JAPAN vol. 016, no. 207 (C-0941), 18 May 1992 (1992-05-18) & JP 04 037660 A (SHIN ETSU CHEM CO LTD), 7 February 1992 (1992-02-07)

## Description

### FIELD OF THE INVENTION

This invention relates to component assemblies and a method of producing a hermetically sealed ceramic to metal bond for implantation in living tissue.

### SUMMARY OF THE INVENTION

The present invention provides component assemblies for use in living tissue as set forth in claims 1, 5 and 10. The invention also provides a method of producing a ceramic to metal bond as set forth in claim 19.

### BRIEF DESCRIPTION OF THE DRAWINGS

**FIG. 1** illustrates a side view of the component assembly with the laminant interlayer material as a foil between the ceramic and metal parts.
**FIG. 2** schematically depicts the bonding steps of the present invention.
**FIG. 3** illustrates the laminant interlayer material layers structure having three foil layers.
**FIG. 4** illustrates the laminant interlayer material layers structure having five foil layers.
**FIG. 5** illustrates the compact interlayer material comprised of material spheres.
**FIG. 6** presents a cross-sectional view of the interlayer material spheres.

### DETAILED DESCRIPTION OF THE PREFERRED EMBODIMENT

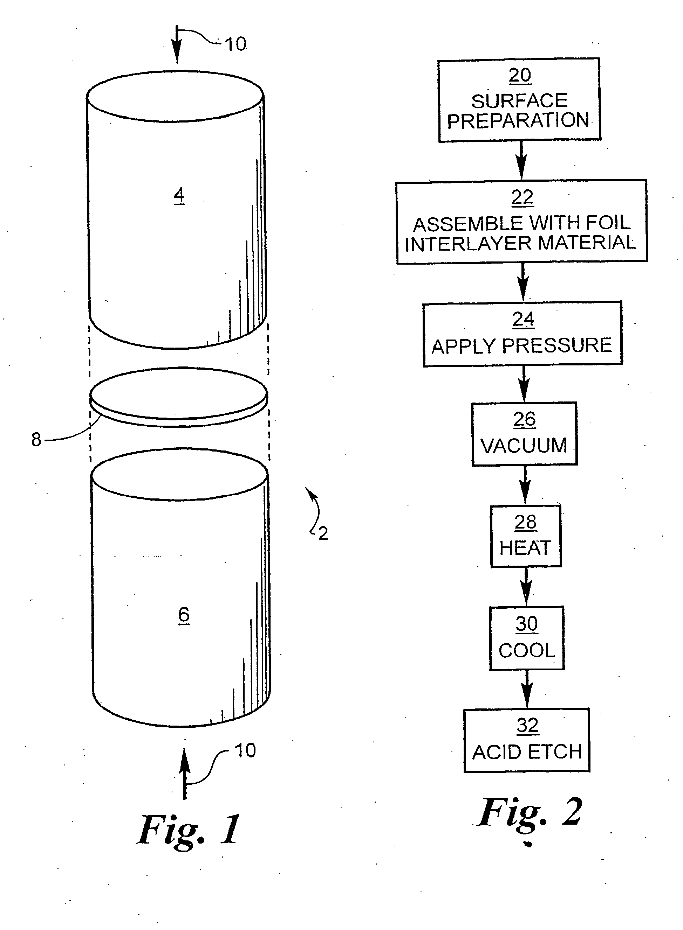

FIG. 1 shows component assembly 2 having metal part 4, ceramic part 6, and laminated interlayer material 8. Component assembly 2 is heated to a specific process temperature, that is below the melting point of metal part 4 or of the ceramic part 6, for a specific period of time, at a pressure that is created by force 10 and that is exerted so as to place laminated interlayer material 8 in intimate contact with the metal and ceramic parts.

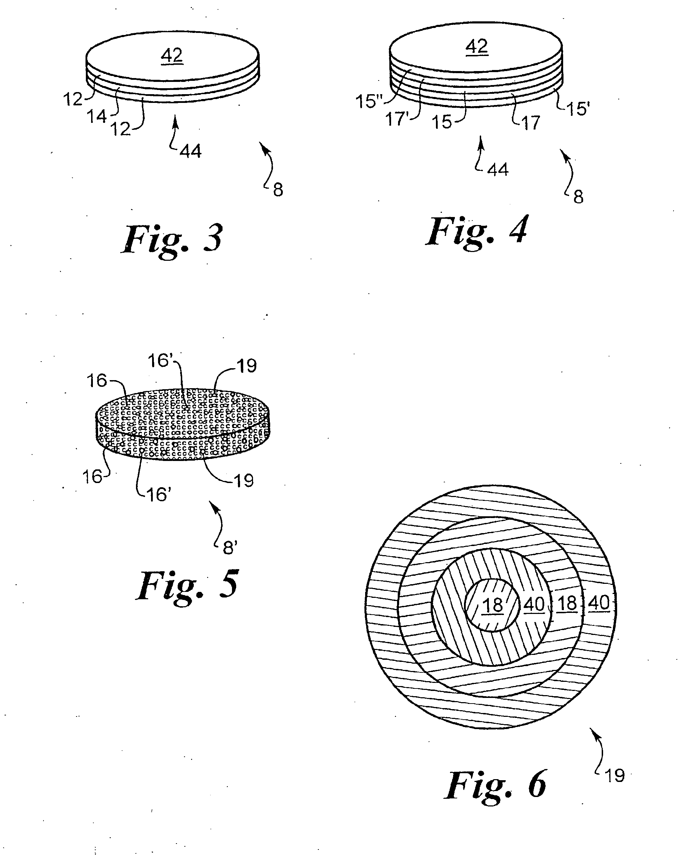

Laminated interlayer material 8 comprises a metal foil having a thickness of about five-thousandths of an inch or less (130µm or less). Laminated interlayer material 8 is a bonded thin stack of metal foil layers selected from the group of materials that are compatible with the ceramic chosen for ceramic part 6 in that they wet the surface during the bonding process and enter into a diffusion process with the ceramic part 6 thereby creating a strong bond joint during processing. The inventors prefer the term "laminated" versus other descriptive, but equally applicable, terms for the invention, such as "clad" material. Laminated interlayer material 8 also is selected from the group of materials that are compatible with the metal chosen for a metal part 4. Laminated interlayer material 8 forms a bond with the metal part 4 by virtue of developing a eutectic alloy at the bonding temperature and pressure utilized during processing. The lowest eutectic temperature, for example, in the nickel-titanium system is about 942°C at about 28 weight percent nickel and 72 weight percent titanium. The group of laminated interlayer materials include substantially pure nickel, i.e., pure nickel and nickel containing approximately two percent or less by weight of other alloy metals and substantially pure titanium, i.e., pure titanium and titanium containing approximately two percent or less by weight of other alloy metals. In a preferred embodiment, **FIG. 3**, laminated interlayer material 8 contains layer 12 comprising commercially pure nickel foil having at least 99.0% nickel and less than 1.0% of other elements with a thickness of approximately 0.0003 inches. Mating layer 14 comprises commercially pure titanium foil having at least 99.0% titanium and less than 1.0% of other elements with a thickness of approximately 0.0003 inches.

Metal part 4 may be selected from a group of biocompatible materials, such as a titanium alloy, and is Ti-6AI-4V in a preferred embodiment. Ceramic part 6 may be alumina, titania, zirconia, stabilized-zirconia, partially-stabilized zirconia, tetragonal zirconia, magnesia-stabilized zirconia, ceria-stabilized zirconia, yttria-stabilized zirconia, and calcia-stabilized zirconia, and in a preferred embodiment ceramic part 6 is yttria-stabilized zirconia. In alternative embodiments, rather than using laminated interlayer material 8 as a foil, interlayer material 8 may be a stack of thin coatings that are applied to either the metal part 4 or ceramic part 6 surface to be bonded by any of a variety of chemical processes such as electroless plating and electroplating, or by any of a variety of thermal processes such as sputtering, evaporating, or ion beam enhanced deposition. Laminated interlayer material 8 may also be applied as layers of thin coatings of metallic beads or metallic powders.

The process steps that are employed to create assembly 2 with a strong bond between metal part 4 and ceramic part 6 are schematically represented in **FIG. 2**. First, the surfaces to be bonded are prepared in step 20 by machining to assure that they will intimately conform to each other during bonding. The surfaces are smoothed and cleaned.

In step 22, component assembly 2 is prepared with laminated interlayer material 8 between metal part 4 and ceramic part 6. In step 24, force 10 is applied to compress laminated interlayer material 8 between metal part 4 and ceramic part 6. Force 10 is sufficient to create intimate contact between the parts. Force 10 is applied to assure that a homogeneous bond is formed between metal part 4 and ceramic part 6, thus creating a hermetic seal between the two parts.

In step 26 the assembly to be heat processed is placed in a furnace in a non-reactive atmosphere, which is preferably vacuum, but which can be argon in an alternative embodiment. A vacuum is applied before the furnace is heated to the processing temperature in step 28. A preliminary holding temperature may be used to allow the thermal mass of the parts to achieve equilibrium before proceeding with heating. The process temperature is lower than the melting point of metal part 4, but greater than the temperature of the eutectic formed by metal 4 and laminated interlayer material 8 (or the eutectic formed within the interlayer or material). It is notable that the laminated interlayer material 8 behaves significantly differently from an alloy of nickel-titanium when heated, as in the application of a braze foil. It is well known that an alloy of nickel-titanium will behave according to the phase diagram relationships that exist for that alloy composition or that exist for that same alloy composition that is heated in contact with a nickel body. On the other hand, for example, heating a pure nickel material in contact with a pure titanium material results in at least some liquidus formation at the lowest eutectic temperature, which is about 942°C (at the eutectic composition of about 28 weight percent nickel and 72 weight percent titanium).

In a preferred embodiment, the vacuum is 10⁻⁶ to 10⁻⁷ torr, to assure that the laminated interlayer material 8 and metal part 4 do not oxidize. Component assembly 2 is held at the selected temperature, which is typically between approximately 942° and 1080°C, for approximately 5 to 20 minutes, while force 10 continues to be exerted on laminated interlayer material 8. The exact time, temperature and pressure are variable with each other so as to achieve a homogeneous and strong bond of metal part 4 with ceramic part 6. For example, in a preferred embodiment, an yttria-stabilized zirconia part bonds to a Ti-6Al-4V part in vacuum at 10⁻⁶ torr at approximately 980°C for 10 minutes with a pressure of approximately 5 to 20 psi on a laminated foil comprised of at least one commercially pure nickel layer of approximately 0.0007 inches thickness and at least one titanium layer of 0.0013 inches, yielding a 50 weight percent nickel and 50 weight percent mean composition titanium laminated interlayer material 8.

The component assembly 2 is furnace cooled to room temperature in step 30. In optional step 32, component assembly 2 is cleaned by being placed in a bath, after thermal processing is complete, to assure removal of all nickel and nickel salts. This bath is preferably an acid bath that etches the exposed surfaces of component assembly 2. In a preferred embodiment, the bath is nitric acid. Removal of nickel and nickel salts in the bath etch insures that component assembly 2 is biocompatible. Nickel and nickel salts are detrimental to living animal tissue. In a preferred embodiment, however, all of the nickel that is introduced as laminated interlayer material 8 is combined with the titanium and is combined chemically to be unavailable as free nickel or as a nickel salt.

**FIG. 3** represents the laminated interlayer material 8 that is preferably comprised of at least two layers of foil each having a thickness of about 0.001 inches. In one embodiment, layer 12 is comprised of a substantially pure nickel while mating layer 14 is comprised of substantially pure titanium. The two layers are in intimate contact and are bonded by conventional processes to perform as a single thin foil. The mean composition of the laminated interlayer material 8 is controlled by adjusting the thickness of layer 12 and layer 14 by well known methods that utilizes the thickness alone to arrive at a volume percentage or alternately that utilizes the density of each material to calculate the required thickness to arrive at a desired weight percentage of each metal. **FIG. 3** illustrates an alternative embodiment where laminated interlayer material 8 is comprised of three layers, and the combination of an odd number of layers results in layer 12 being on both the top outer surface 42 and the bottom outer surface 44.

In yet a further embodiment, illustrated in **FIG. 4**, multiple layers of laminating metals may be chosen of varying thicknesses to effect a desired property in the laminated interlayer material 8. It is preferred that the mean composition of the laminated interlayer material 8 be chosen so that depletion of the material, nickel or titanium, for example, does not occur during the brazing operation. Preferred mean compositions for laminated interlayer material 8 contain about 20 to 70 weight percent of nickel with the balance titanium. For example, a five layer laminated interlayer material 8 may be comprised of a mean composition of 50 weight percent nickel and 50 weight percent titanium, where both the top outer surface 42 and the bottom outer surface 44 are metal foil layers 15' and 15", preferably comprised of nickel, the middle foil layer 15 is comprised of nickel, and the two inner mating foil layers 17 and 17', on either side of the middle foil layer 15, are comprised of titanium. The total thickness of all of the nickel layers and the total thickness of both of the titanium layers being equal to each other, thereby maintaining the 50:50 volume percentages. However, if it is desired to have more nickel available for bonding to the ceramic part 6 or to the metal part 4 on either the top outer surface 42 or the bottom outer surface 44 of the laminated interlayer 8, then either or both of the metal foil layers 15' and/or 15" may be preferentially thickened at the expense of the middle metal foil layer 15, thereby maintaining the desired overall mean composition of 50 volume percent nickel and 50 volume percent titanium.

In a preferred embodiment, the top outer surface 42 and the bottom outer surface 44 are nickel. The nickel layer contacts the titanium metal part thereby facilitating the forming of a nickel-titanium eutectic alloy.

In this embodiment, a compact interlayer material 8', illustrated in **FIG. 5**, is comprised of a multitude of smell spherical metal composite particles, each having a controlled composition. For example, spheres of two distinct compositions, primary alloy sphere 16, which may be nickel, and secondary alloy sphere 16', which may be titanium, are uniformly combined into a homogeneous compact that sinters into a dense compact at temperature and optionally under pressure, as previously illustrated with force 10 of **FIG. 1**. Obviously, there may be more than two sets of spheres having different compositions or morphologies, but these alternatives are not illustrated. It is known that the small particles may have other shapes than spheres and that they may not be uniform in size or shape. Further, the final composition of the compact interlayer material 8' is controlled by total volume of primary alloy sphere 16 and secondary alloy sphere 16' and as the compact interlayer material 8' has been additionally alloyed by diffusion from metal part 4 and/or ceramic part 6 of **FIG. 1**. It is preferred that the mean composition of the compact interlayer material 8' be chosen so that depletion of either or any of the materials, nickel or titanium, for example, does not occur during the brazing operation. Preferred mean compositions for compact interlayer material 8' contain about 20 to 70 volume percent of nickel with the balance titanium.

In a further alternative embodiment, the primary alloy sphere 16 or the secondary alloy sphere 16' may be comprised of layered materials, as illustrated by composite sphere 19, **FIG. 6**, wherein the sets of spheres, illustrated in **FIG. 5**, may have the same composition or they may have different compositions of layered materials. In a preferred embodiment, for example, composite sphere 19 presents the cross-sectional view of a layered composite sphere that is comprised of primary sphere laminate layer 18, optionally titanium, alternated with secondary sphere laminate layer 40, optionally nickel. Compact interlayer material 8' is comprised of a plurality of composite spheres 19, wherein the distribution of sizes, morphologies, and shapes is varied to achieve the desired bond between metal part 4 and ceramic part 6 when temperature and optionally pressure are applied.

As previously discussed, but equally applicable to the invention, it is preferred that the mean composition of the compact interlayer material 8' be chosen so that depletion of the material, nickel or titanium, for example, does not occur during the brazing operation. Preferred mean compositions for compact interlayer material 8' contain about 20 to 70 volume percent of nickel with the balance titanium. It is preferred that the mean composition of primary alloy sphere 16 of **FIG. 6** comply with this compositional guideline. While numerous metals demonstrate formation of eutectic compositions, nickel and titanium are exemplar selections that are useful in an implantable device. Other interesting materials that demonstrate eutectic formation include nickel-titanium, titanium-copper-silver, titanium-copper-nickel, gold-tin, copper-silver, copper-magnesium, copper-titanium, niobium-nickel, nickel-silicon, nickel-zirconium, silver-silicon, silver-tin, silver-titanium, gold-silicon, and gold-titanium.

The layered spheres 19 are made by any of a number of known techniques, including aerosol techniques or vapor deposition techniques. In this manner, the total composition of the compact interlayer material is controllable on a macro level as well as on a micro (intersphere) level, thereby aiding densification of the compact interlayer material 8' and the bonding of component assembly 2. It is known to the inventor to vary the composition of the outer layer of the secondary sphere laminate layer 40, as illustrated in **FIG. 6**, to enhance densification and bonding at low temperatures and, optionally, to enhance bonding at low pressure/forces 10.

Component assembly 2 is preferably biocompatible after bonding and processing. Metal part 4, ceramic part 6, and laminated interlayer material 8 are selected to be compatible with the environment in a living body. Hence, metal part 4 is typically a titanium alloy and ceramic part 6 is typically zirconia.

In a preferred embodiment, component assembly 2 is either an electrical sensor or an electrical stimulator that is implanted in a human body, although it could equally well be implanted in any animal. It must survive long periods in the hostile environment of a living body, which is basically a warm saline solution. In a preferred embodiment, component assembly 2 is either a sensor or stimulator comprised of a hollow ceramic tube that contains various electronic components that is bonded to a metal electrode end. The component assembly is preferably watertight; hence, the bond is hermetic, resisting salt-water intrusion as well as growth of living tissue into the metal-to-ceramic bond joint.

Further, component assembly 2 does not corrode while implanted in the body. The materials are chosen such that post-bonding they are not susceptible to corrosion either individually or in the as-bonded state. Component assembly 2 resists electrolytic corrosion as well as crevice corrosion, because of the materials selected for component assembly 2.

Obviously, many modifications and variations of the present invention are possible in light of the above teachings. It is therefore to be understood that, within the scope of the appended claims, the invention may be practiced otherwise than as specifically described.

## Claims

1. A component assembly (2) for use in living tissue comprising:
a ceramic part (6);
a metal part (4); and
a compact interlayer material (8') comprised of at least one set of spherical metal composite particles (19), said at least one set of metal composite particles (19) comprised of at least one primary particle laminate layer (18) and at least one secondary particle laminate layer (40), each layer comprising a metal selected from the group consisting of metals that form a eutectic composition, for bonding said ceramic part (6) to said metal part (4).

2. The component assembly (2) of claim 1 wherein said at least one primary particle laminate layer (18) is comprised of substantially pure nickel and said at least one secondary particle laminate layer (40) is comprised of substantially pure titanium.

3. The component assembly (2) of claim 1 wherein said at least one primary sphere laminate layer (18) is comprised of substantially pure nickel and said at least one secondary sphere laminate layer (40) is comprised of an alloy of titanium.

4. The component assembly (2) of any one of the preceding claims wherein said at least one set of composite particles (19) has an outer layer that is comprised of substantially pure nickel.

5. A component assembly (2) for use in living tissue comprising:
a ceramic part (6);
a metal part (4); and
a compact interlayer material (8') comprised of at least two sets of spherical metal particles (16, 16'), each set comprised of a metal selected from a group of metals that form a eutectic composition, for bonding said ceramic part (6) to said metal part (4).

6. The component assembly (2) of claim 5 wherein said at least two sets of metal particles are comprised of substantially pure metals.

7. The component assembly (2) of claim 5 or claim 6 wherein said compact interlayer material (8') is comprised of a set of primary metal particles comprised of nickel particles and a set of secondary metal particles comprised of titanium particles for bonding said ceramic part (6) to said metal part (4).

8. The component assembly (2) of claim 7 wherein said nickel particles and said titanium particles are approximately spherical in shape.

9. The component assembly (2) of any one of claims 1 to 7 wherein the metal particles are spheres.

10. A component assembly (2) for use in living tissue comprising:
a ceramic part (6);
a metal part (4); and
a compact interlayer material (8) comprised of at least three metal laminate layers (15, 17, 15', 17'), each layer comprising a metal selected from the group consisting of metals that form a eutectic composition, for bonding said ceramic part (6) to said metal part (4).

11. The component assembly of claim 10 wherein at least one of said at least three laminate layers is substantially pure nickel and another of said layers is substantially pure titanium.

12. The component assembly of claims 10 or claim 11 wherein said interlayer material is a laminated metal foil.

13. The component assembly of claim 10 or claim 11 wherein said interlayer material comprises layers of thin coatings of metallic beads or metallic powders.

14. The component assembly (2) of any one of the preceding claims wherein said ceramic part (6) is selected from the group consisting of alumina, titania, zirconia, stabilized-zirconia, partially-stabilized zirconia, tetragonal zirconia, magnesia-stabilized zirconia, ceria-stabilized zirconia, yttria-stabilized zirconia, calcia-stabilized zirconia, and yttria-stabilized zirconia.

15. The component assembly (2) of any one of the preceding claims wherein said metal part (4) is selected from the group consisting of titanium and titanium alloys.

16. The component assembly (2) of any one of the preceding claims wherein said compact interlayer material (8') reacts with and forms a bond between said ceramic part (6) and said metal part (4).

17. The component assembly (2) of any one of the preceding claims wherein said compact interlayer material (8') has a thickness of approximately 0.005 inches or less (1300µm or less).

18. The component assembly of any one of the preceding claims wherein said assembly is (2) is heated to a temperature that is less than the melting point of said metal part (4) but that is greater than the eutectic temperature of said component interlayer material (8') and said metal part (4), thereby forming a bond.

19. A method of producing a hermetically sealed ceramic to metal bond for implantation in human tissue, the method comprising the steps of:
selecting a ceramic part (6);
selecting a metal part (4);
selecting a compact interlayer material (8), said interlayer material being comprised of:
a) at least one set of metal composite particles (19), said at least one set of metal composite particles (19) comprised of at least one primary particle laminate layer (18) and at least one secondary particle laminate layer (40), each layer comprising a metal selected from the group consisting of metals that form a eutectic composition, for bonding said ceramic part (6) to said metal part (4); or
b) at least two sets of metal particles (16, 16'), each set comprised of a metal selected from a group of metals that form a eutectic composition, for bonding said ceramic part (6) to said metal part (4); or
c) at least three metal laminate layers, each layer comprising a metal selected from the group consisting of metals that form a eutectic composition, for bonding said ceramic part (6) to said metal part (4);
interposing said interlayer material (8) between said ceramic part (6) and said metal part (4);
applying a force to said ceramic part and said metal part to place said interposed interlayer material in compression;
placing assembly of the ceramic and metal parts and interlayer material in a non-reactive atmosphere;
heating said assembly to a process temperature lower than the melting point of metal part (4) but greater than the eutectic temperature of said component interlayer material (8') and said metal part (4);
holding said assembly at said process temperature for a predetermined time to form a bond between said ceramic part (6) and said metal part (4); and
cooling the assembly.

## Patentansprüche

1. Komponentenanordnung (2) zur Verwendung in Lebendgewebe, die Folgendes umfasst:
ein Keramikteil (6),
ein Metallteil (4) und
ein kompaktes Zwischenschichtmaterial (8') aus zumindest einem Satz kugelförmiger Metallverbundteilchen (19), wobei der zumindest eine Satz von Metallverbundteilchen (19) aus zumindest einer Primärteilchenlaminatschicht (18) und zumindest einer Sekundärteilchenlaminatschicht (40) besteht, wobei jede Schicht ein Metall umfasst, das aus der aus eutektische Zusammensetzungen bildenden Metallen bestehenden Gruppe ausgewählt ist, um das Keramikteil (6) haftschlüssig mit dem Metallteil (4) zu verbinden.

2. Komponentenanordnung (2) nach Anspruch 1, worin die zumindest eine Kugel-Primärteilchenlaminatschicht (18) aus im Wesentlichen reinem Nickel besteht und die zumindest eine Kugel-Sekundärteilchenlaminatschicht (40) aus im Wesentlichen reinem Titan besteht.

3. Komponentenanordnung (2) nach Anspruch 1, worin die zumindest eine Primärteilchenlaminatschicht (18) aus im Wesentlichen reinem Nickel besteht und die zumindest eine Sekundärteilchenlaminatschicht (40) aus einer Titanlegierung besteht.

4. Komponentenanordnung (2) nach einem der vorangegangenen Ansprüche, worin der zumindest eine Satz von Verbundteilchen (19) eine Außenschicht aufweist, die aus im Wesentlichen reinem Nickel besteht.

5. Komponentenanordnung (2) zur Verwendung in Lebendgewebe, die Folgendes umfasst:
ein Keramikteil (6),
ein Metallteil (4) und
ein kompaktes Zwischenschichtmaterial (8') aus zumindest zwei Sätzen kugelförmiger Metallverbundteilchen (16, 16'), wobei jeder Satz aus einem Metall besteht, das aus der aus eutektische Zusammensetzungen bildenden Metallen bestehenden Gruppe ausgewählt ist, um das Keramikteil (6) haftschlüssig mit dem Metallteil (4) zu verbinden.

6. Komponentenanordnung (2) nach Anspruch 5, worin die zumindest zwei Sätze von Metallteilchen aus im Wesentlichen reinen Metallen bestehen.

7. Komponentenanordnung (2) nach Anspruch 5 oder 6, worin das kompakte Zwischenschichtmaterial (8') aus einem Satz Primärmetallteilchen, die aus Nickelteilchen bestehen, und einem Satz Sekundärmetallteilchen, die aus Titanteilchen bestehen, besteht, um das Keramikteil (6) haftschlüssig mit dem Metallteil (4) zu verbinden.

8. Komponentenanordnung (2) nach Anspruch 7, worin die Nickelteilchen und die Titanteilchen etwa kugelförmig sind.

9. Komponentenanordnung (2) nach einem der Ansprüche 1 bis 7, worin die Metallteilchen Kügelchen sind.

10. Komponentenanordnung (2) zur Verwendung in Lebendgewebe, die Folgendes umfasst:
ein Keramikteil (6),
ein Metallteil (4) und
ein kompaktes Zwischenschichtmaterial (8) aus zumindest drei Metalllaminatschichten (15, 17, 15', 17'), die jeweils aus einem Metall bestehen, das aus der aus eutektische Zusammensetzungen bildenden Metallen bestehenden Gruppe ausgewählt ist, um das Keramikteil (6) haftschlüssig mit dem Metallteil (4) zu verbinden.

11. Komponentenanordnung nach Anspruch 10, worin zumindest eine der zumindest drei Laminatschichten im Wesentlichen reiner Nickel ist und eine weitere der Schichten im Wesentlichen reines Titan ist.

12. Komponentenanordnung nach Anspruch 10 oder 11, worin das Zwischenschichtmaterial eine laminierte Metallfolie ist.

13. Komponentenanordnung nach Anspruch 10 oder 11, worin das Zwischenschichtmaterial Schichten dünner Beschichtungen aus Metallperlen oder Metallpulvern umfasst.

14. Komponentenanordnung (2) nach einem der vorangegangenen Ansprüche, worin das Keramikteil (6) aus der aus folgenden bestehenden Gruppe ausgewählt ist: Aluminiumdioxid, Titandioxid, Zirkoniumoxid, stabilisiertem Zirkoniumoxid, teilweise stabilisiertem Zirkoniumoxid, tetragonalem Zirkoniumoxid, mit Magnesiumoxid stabilisiertem Zirkoniumoxid, mit Cerdioxid stabilisiertem Zirkoniumoxid, mit Yttriumoxid stabilisiertem Zirkoniumoxid, mit Calciumoxid stabilisiertem Zirkoniumoxid und mit Yttriumoxid stabilisiertem Zirkoniumoxid.

15. Komponentenanordnung (2) nach einem der vorangegangenen Ansprüche, worin das Metallteil (4) aus der aus Titan und Titanlegierungen bestehenden Gruppe ausgewählt ist.

16. Komponentenanordnung (2) nach einem der vorangegangenen Ansprüche, worin das kompakte Zwischenschichtmaterial (8') mit dem Keramikteil (6) und dem Metallteil (4) reagiert und eine haftschlüssige Verbindung zwischen diesen bildet.

17. Komponentenanordnung (2) nach einem der vorangegangenen Ansprüche, worin das kompakte Zwischenschichtmaterial (8') eine Dicke von etwa 0,005 Zoll oder weniger (130 µm oder weniger) aufweist.

18. Komponentenanordnung (2) nach einem der vorangegangenen Ansprüche, worin diese auf eine Temperatur erhitzt wird, die geringer ist als der Schmelzpunkt des Metallteils (4), aber höher als die eutektische Temperatur des Komponentenzwischenschichtmaterials (8') und des Metallteils (4), wodurch eine haftschlüssige Verbindung gebildet wird.

19. Verfahren zur Herstellung einer hermetisch abgedichteten haftschlüssigen Keramik-Metall-Verbindung zur Implantation in menschliches Gewebe, wobei das Verfahren folgende Schritte umfasst:
die Auswahl eines Keramikteils (6),
die Auswahl eines Metallteils (4),
die Auswahl eines kompakten Zwischenschichtmaterials (8), wobei das Zwischenschichtmaterial aus Folgendem besteht:
a) zumindest einem Satz Metallverbundteilchen (19), wobei der zumindest eine Satz von Metallverbundteilchen (19) aus zumindest einer Primärteilchenlaminatschicht (18) und zumindest einer Sekundärteilchenlaminatschicht (40) besteht, wobei jede Schicht ein Metall umfasst, das aus der aus eutektische Zusammensetzungen bildenden Metallen bestehenden Gruppe ausgewählt ist, um das Keramikteil (6) haftschlüssig mit dem Metallteil (4) zu verbinden; oder
b) zumindest zwei Sätzen Metallverbundteilchen (16, 16'), wobei jeder Satz aus einem Metall besteht, das aus der aus eutektische Zusammensetzungen bildenden Metallen bestehenden Gruppe ausgewählt ist, um das Keramikteil (6) haftschlüssig mit dem Metallteil (4) zu verbinden; oder
c) zumindest drei Metalllaminatschichten, die jeweils aus einem Metall bestehen, das aus der aus eutektische Zusammensetzungen bildenden Metallen bestehenden Gruppe ausgewählt ist, um das Keramikteil (6) haftschlüssig mit dem Metallteil (4) zu verbinden;
das Anordnen des Zwischenschichtmaterials (8) zwischen dem Keramikteil (6) und dem Metallteil (4);
das Ausüben einer Kraft auf das Keramikteil und den Metallteil, um das dazwischen angeordnete Zwischenschichtmaterial zu komprimieren;
das Platzieren der Anordnung aus dem Keramik- und dem Metallteil und dem Zwischenschichtmaterial in einer nicht reaktiven Atmosphäre;
das Erhitzen der Anordnung auf eine Verfahrenstemperatur, die geringer ist als der Schmelzpunkt des Metallteils (4), aber höher als die eutektische Temperatur des Komponentenzwischenschichtmaterials (8') und des Metallteils (4);
das Halten der Anordnung auf dieser Verfahrenstemperatur für eine vorbestimmte Dauer, um eine haftschlüssige Verbindung zwischen dem Keramikteil (6) und dem Metallteil (4) zu bilden, und
das Abkühlen der Anordnung.

## Revendications

1. Assemblage de composants (2) pour utilisation dans un tissu vivant, comprenant :
une partie en céramique (6) ;
une partie en métal (4) ; et
un matériau de couche intermédiaire compact (8') constitué d'au moins un jeu de particules composites métalliques sphériques (19), ledit au moins un jeu de particules composites métalliques (19) étant constitué d'au moins une couche stratifiée de particules primaires (18) et d'au moins une couche stratifiée de particules secondaires (40), chaque couche comprenant un métal choisi dans le groupe constitué par les métaux qui forment une composition eutectique, pour coller ladite partie en céramique (6) à ladite partie en métal (4).

2. Assemblage de composants (2) selon la revendication 1, dans lequel ladite au moins une couche stratifiée de particules primaires (18) est constituée de nickel pratiquement pur et ladite au moins une couche stratifiée de particules secondaires (40) est constituée de titane pratiquement pur.

3. Assemblage de composants (2) selon la revendication 1, dans lequel ladite au moins une couche stratifiée de sphères primaires (18) est constituée de nickel pratiquement pur et ladite au moins une couche stratifiée de sphères secondaires (40) est constituée d'un alliage de titane.

4. Assemblage de composants (2) selon l'une quelconque des revendications précédentes, dans lequel ledit au moins un jeu de particules composites (19) a une couche extérieure qui est constituée de nickel pratiquement pur.

5. Assemblage de composants (2) pour utilisation dans un tissu vivant, comprenant :
une partie en céramique (6) ;
une partie en métal (4) ; et
un matériau de couche intermédiaire compact (8') constitué d'au moins deux jeux de particules métalliques sphériques (16, 16'), chaque jeu étant constitué d'un métal choisi dans le groupe constitué par les métaux qui forment une composition eutectique, pour coller ladite partie en céramique (6) à ladite partie en métal (4).

6. Assemblage de composants (2) selon la revendication 5, dans lequel lesdits au moins deux jeux de particules métalliques sont constitués de métaux pratiquement purs.

7. Assemblage de composants (2) selon la revendication 5 ou la revendication 6, dans lequel ledit matériau de couche intermédiaire compact (8') est constitué d'un jeu de particules métalliques primaires constituées de particules de nickel et d'un jeu de particules métalliques secondaires constituée de particules de titane pour coller ladite partie en céramique (6) à ladite partie en métal (4).

8. Assemblage de composants (2) selon la revendication 7, dans lequel lesdites particules de nickel et lesdites particules de titane ont une forme approximativement sphérique.

9. Assemblage de composants (2) selon l'une quelconque des revendications 1 à 7, dans lequel les particules métalliques sont des sphères.

10. Assemblage de composants (2) pour utilisation dans un tissu vivant, comprenant :
une partie en céramique (6) ;
une partie en métal (4) ; et
un matériau de couche intermédiaire compact (8) constitué d'au moins trois couches stratifiées métalliques (15, 17, 15', 17'), chaque couche comprenant un métal choisi dans le groupe constitué par les métaux qui forment une composition eutectique, pour coller ladite partie en céramique (6) à ladite partie en métal (4).

11. Assemblage de composants selon la revendication 10, dans lequel au moins l'une desdites au moins trois couches stratifiées est en nickel pratiquement pur et une autre desdites couches est en titane pratiquement pur.

12. Assemblage de composants selon la revendication 10 ou la revendication 11, dans lequel ledit matériau de couche intermédiaire est une feuille métallique stratifiée.

13. Assemblage de composants selon la revendication 10 ou la revendication 11, dans lequel ledit matériau de couche intermédiaire comprend des couches de revêtements minces de perles métalliques ou de poudres métalliques.

14. Assemblage de composants (2) selon l'une quelconque des revendications précédentes, dans lequel ladite partie en céramique (6) est choisie dans le groupe constitué par l'alumine, l'oxyde de titane, la zircone, la zircone stabilisée, la zircone partiellement stabilisée, la zircone tétragonale, la zircone stabilisée à la magnésie, la zircone stabilisée à l'oxyde de cérium, la zircone stabilisée à l'oxyde d'yttrium, la zircone stabilisée à l'oxyde de calcium, et la zircone stabilisée à l'oxyde d'yttrium.

15. Assemblage de composants (2) selon l'une quelconque des revendications précédentes, dans lequel ladite partie en métal (4) est choisie dans le groupe constitué par le titane et les alliages de titane.

16. Assemblage de composants (2) selon l'une quelconque des revendications précédentes, dans lequel ledit matériau de couche intermédiaire compact (8') réagit avec et forme une liaison entre ladite partie en céramique (6) et ladite partie en métal (4).

17. Assemblage de composants (2) selon l'une quelconque des revendications précédentes, dans lequel ledit matériau de couche intermédiaire compact (8') a une épaisseur d'environ 130 µm (0,005 pouce) ou moins.

18. Assemblage de composants (2) selon l'une quelconque des revendications précédentes, lequel assemblage (2) est chauffé à une température qui est inférieure au point de fusion de ladite partie en métal (4) mais est supérieure à la température eutectique dudit matériau de couche intermédiaire constitutif (8') et de ladite partie en métal (4), en formant ainsi une liaison.

19. Procédé pour produire une liaison hermétiquement scellée entre une céramique et un métal pour implantation dans un tissu humain, le procédé comprenant les étapes consistant à
sélectionner une partie en céramique (6) ;
sélectionner une partie en métal (4) ;
sélectionner un matériau de couche intermédiaire compact (8), ledit matériau de couche intermédiaire compact étant constitué de :
a) au moins un jeu de particules composites métalliques (19), ledit au moins un jeu de particules composites métalliques (19) étant constitué d'au moins une couche stratifiée de particules primaires (18) et d'au moins une couche stratifiée de particules secondaires (40), chaque couche comprenant un métal choisi dans le groupe constitué par les métaux qui forment une composition eutectique, pour coller ladite partie en céramique (6) à ladite partie en métal (4) ; ou
b) au moins deux jeux de particules métalliques (16, 16'), chaque jeu étant constitué d'un métal choisi dans le groupe constitué par les métaux qui forment une composition eutectique, pour coller ladite partie en céramique (6) à ladite partie en métal (4) ; ou
c) au moins trois couches stratifiées métalliques, chaque couche comprenant un métal choisi dans le groupe constitué par les métaux qui forment une composition eutectique, pour coller ladite partie en céramique (6) à ladite partie en métal (4) ;
interposer ledit matériau de couche intermédiaire (8) entre ladite partie en céramique (6) et ladite partie en métal (4) ;
appliquer une force à ladite partie en céramique et à ladite partie en métal pour placer ledit matériau de couche intermédiaire interposé en compression ;
placer l'assemblage des parties en céramique et en métal et du matériau de couche intermédiaire dans une atmosphère non réactive ;
chauffer ledit assemblage à une température de traitement inférieure au point de fusion de la partie en métal (4) mais supérieure à la température eutectique dudit matériau de couche intermédiaire constitutif (8') et de ladite partie en métal (4);
maintenir ledit assemblage à ladite température de traitement pendant un temps prédéterminé pour former une liaison entre ladite partie en céramique (6) et ladite partie en métal (4) ; et
refroidir l'assemblage.
